# EUROPEAN PATENT APPLICATION

(11) **EP 4 483 930 A1**
(43) Date of publication of application: **01.01.2025**
(21) Application number: 22944205.8
(22) Date of filing: 31.05.2022
(51) Int. Cl.: A61M 16/00

(54) **METHOD FOR CONTROLLING VENTILATION APPARATUS, VENTILATION SYSTEM AND VENTILATION APPARATUS**

(71) Applicant: Shenzhen Mindray Bio-Medical Electronics Co., Ltd, Shenzhen, Guangdong 518057 (CN)
(72) Inventor: LIU, Jinglei, Shenzhen, Guangdong 518057 (CN); ZHOU, Xiaoyong, Shenzhen, Guangdong 518057 (CN); HUANG, Zhiwen, Shenzhen, Guangdong 518057 (CN); WANG, Jianxin, Shenzhen, Guangdong 518057 (CN)
(74) Representative: KIPA AB
(86) International application number: PCT/CN2022/096321
(87) International publication number: WO 2023/230868

(57) **Abstract**

A method (100) for controlling a ventilation apparatus, a ventilation system, and a ventilation apparatus. The method (100) comprises: acquiring physiological state parameters representing the physiological states of a ventilation object, the physiological state parameters including ventilation-related monitoring parameters (S 110); acquiring a ventilation target for providing mechanical ventilation for the ventilation object (S120); and on the basis of the physiological state parameters and the ventilation target, setting the intensity of a diaphragm pacing stimulation signal sent to a target position of the ventilation object, and setting a ventilation parameter for mechanical ventilation of the ventilation object, so as to perform diaphragm pacing and mechanical ventilation on the ventilation object (S130). The synergistic control of diaphragm pacing and mechanical ventilation can reduce or avoid lung injury or circulatory depression caused by mechanical ventilation, and prevent diaphragm disuse atrophy.

## Description

### TECHNICAL FIELD

The disclosure relates to a technical field of medical apparatus, and more particularly to a method for controlling a ventilation apparatus, a ventilation system, and a ventilation apparatus.

### BACKGROUND

Mechanical ventilation therapy is often used to provide ventilation support for a patient who is critically ill or undergoes anesthesia and surgery. For a critically ill patient, a respiratory failure is the most common organ failure, which seriously threatens life safety of the patient. For a patient who undergoes anesthesia and surgery, as the patient loses a spontaneous ability, a mechanical ventilation support is still required.

Current mechanical ventilation is mainly positive pressure ventilation. When a healthy person inhales, respiratory muscles are contracted to decrease a thoracic pressure, which in turn decreases a pressure in lungs to below an atmospheric pressure. At this time, a gas in atmosphere can enter the lungs to form inhalation. When a healthy person exhales, respiratory muscles are relaxed, and a pressure inside lungs becomes greater than the atmospheric pressure, which enables exhalation. While on the contrary, positive pressure ventilation mainly uses a ventilation apparatus, such as a ventilator or an anesthesia machine, to provide all or part of a respiratory drive force. During inhalation, gas is delivered out through a valve, and an airway pressure is greater than a pressure inside lungs, so that the gas can enter into the lungs. During exhalation, gas is released through a valve, and the airway pressure is less than the pressure in the lungs, so that the gas can be exhaled.

Due to the anti-physiological characteristic of the positive pressure ventilation, while providing ventilation support to a patient, certain damages are caused to the respiratory and circulatory system of the patient. For example, excessive positive pressure ventilation may cause pulmonary pressure injury, and excessive tidal volume may cause volumetric injury. As the positive pressure ventilation causes an increased intrathoracic pressure, which compresses heart, thus causing an increased right cardiac preload, which in turn affects cardiac output and leads to circulatory inhibition. Long-term mechanical ventilation can also lead to disuse atrophy of a respiratory muscle, such as diaphragm, making it difficult for the patient to get off the ventilator.

Diaphragm pacing technology is an invasive or non-invasive way to electrically stimulate phrenic nerve or diaphragm to increase an activity of the diaphragm for improving respiration. This treatment is mainly used in a rehabilitation department, such as for a patient with spinal cord injuries. However, it cannot provide an oxygen support or positive end-expiratory pressure (PEEP) support to a critically ill patient or anesthetized patient.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides a method for controlling a ventilation apparatus, including:
acquiring a physiological state parameter which represents a physiological state of a ventilated object, wherein the physiological state parameter includes a monitoring parameter which is related to ventilation;
acquiring a ventilation target for providing mechanical ventilation to the ventilated object; and
setting an intensity of a stimulation signal for diaphragm pacing which signal is transmitted to a target position of the ventilated object and setting a ventilation parameter for the mechanical ventilation of the ventilated object, based on the physiological state parameter and the ventilation target, so as to perform the diaphragm pacing and the mechanical ventilation on the ventilated object.

A second aspect according to an embodiment of this disclosure provides a ventilation system, including a ventilation apparatus, which includes:
a sensor, which is configured to acquire a monitoring parameter of a ventilated object, which parameter is related to ventilation;
a respiratory loop, which is configured to deliver a gas which is provided by a gas source to the ventilated object for mechanical ventilation;
a diaphragm pacing apparatus, which is configured to contact a target position of the ventilated object through a stimulation electrode, and transmit a stimulation signal to the target position, so as to perform diaphragm pacing on the ventilated object; and
a processor, which is connected with the ventilation apparatus and the diaphragm pacing apparatus, and configured to set an intensity of the stimulation signal for the diaphragm pacing and a ventilation parameter for the mechanical ventilation.

A third aspect according to an embodiment of this disclosure provides a ventilation apparatus, including:
a sensor, which is configured to acquire a monitoring parameter of a ventilated object, which parameter is related to ventilation;
a respiratory loop, which is configured to deliver a gas which is provided by a gas source to the ventilated object for mechanical ventilation;
a communication module, which is capable of being in communicative connection with a diaphragm pacing apparatus; wherein the diaphragm pacing apparatus is configured to contact a target position of the ventilated object through a stimulation electrode, and transmit a stimulation signal to the target position, so as to perform diaphragm pacing on the ventilated object; and
a processor, which is configured to control a ventilation parameter for the mechanical ventilation and an intensity of the stimulation signal for the diaphragm pacing.

According to the method for controlling a ventilation apparatus, the ventilation system, and the ventilation apparatus according to embodiments of this disclosure, positive pressure ventilation of the ventilation apparatus is combined with negative pressure ventilation which is generated by diaphragm pacing, such that a pressure of positive pressure ventilation during mechanical ventilation can be reduced, so as to reduce a thoracic pressure, reduce or avoid lung injury or circulatory inhibition which is caused by mechanical ventilation, and meanwhile the diaphragm is simultaneously stimulated to contract, so as to reduce diaphragm disuse atrophy and avoid dependence on the ventilation apparatus.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be acquired based on these drawings without any creative effort.
FIG. 1 is a schematic flow chart of a method for controlling a ventilation apparatus according to an embodiment of this disclosure.
FIG. 2 is a block diagram of a ventilation system according to an embodiment of this disclosure.
FIG. 3 is a block diagram of a ventilation apparatus according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments acquired by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to thoroughly understand this disclosure, a detailed structure is presented in the following description to illustrate the technical solution proposed in this disclosure. Optional embodiments of this disclosure are described in detail below, however, in addition to these detailed descriptions, this disclosure may also have other implementations.

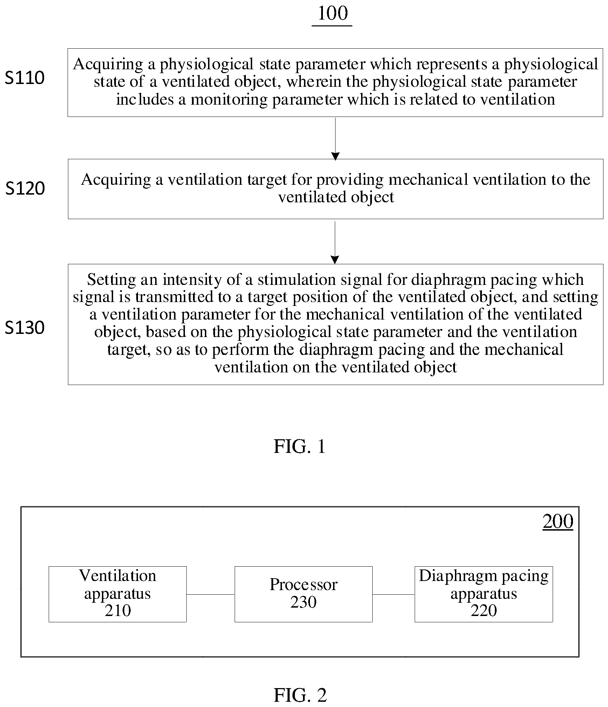

Hereinafter, a method for controlling a ventilation apparatus according to an embodiment of this disclosure is described with reference to FIG. 1. FIG. 1 is a schematic flow chart of a method 100 for controlling a ventilation apparatus according to an embodiment of this disclosure.

As shown in FIG. 1, a method 100 for controlling a ventilation apparatus according to an embodiment of this disclosure includes the following steps.

In step S110, acquire a physiological state parameter which represents a physiological state of a ventilated object, wherein the physiological state parameter includes a monitoring parameter which is related to ventilation;
In step 120, acquire a ventilation target for providing mechanical ventilation to the ventilated object.

In step 130, set an intensity of a stimulation signal for diaphragm pacing which signal is transmitted to a target position of the ventilated object, and set a ventilation parameter for the mechanical ventilation of the ventilated object, based on the physiological state parameter and the ventilation target, so as to perform the diaphragm pacing and the mechanical ventilation on the ventilated object.

According to the method 100 for controlling a ventilation apparatus according to an embodiment of this disclosure, positive pressure ventilation of the mechanical ventilation is combined with negative pressure ventilation which is generated by diaphragm pacing, through controlling an intensity of a stimulation signal for diaphragm pacing and a ventilation parameter for mechanical ventilation, such that a pressure of positive pressure ventilation during mechanical ventilation can be reduced, so as to reduce a thoracic pressure, reduce or avoid lung injury or circulatory inhibition which is caused by mechanical ventilation, and meanwhile the diaphragm is simultaneously stimulated to contract, so as to reduce diaphragm disuse atrophy and avoid dependence on the ventilation apparatus.

Specifically, the mechanical ventilation is provided by a ventilation apparatus, which can be implemented as a medical apparatus with a mechanical ventilation function, such as a ventilator, an anesthesia machine, an oxygen therapy machine, etc. The ventilated object who receives mechanical ventilation and diaphragmatic pacing may specifically refer to an injured or sick patient who requires respiratory assist due to respiratory failure or difficulty in spontaneous respiration. Exemplarily, the ventilation apparatus at least includes a sensor and a respiratory loop, the sensor is configured to acquire a monitoring parameter which is related to ventilation, and the respiratory loop is configured to deliver a gas which is provided by a gas source to a ventilated object for mechanical ventilation. The gas provided by the gas source can be air or a mixture of air and oxygen.

Diaphragm pacing is provided by a diaphragm pacing apparatus, which can be implemented as a module of the ventilation apparatus. For example, the diaphragm pacing apparatus can be a plug-in module, an external module, or a built-in integrated module, of the ventilation apparatus. Diaphragm pacing apparatus can also be implemented as an independent apparatus. In some embodiments, the diaphragm pacing apparatus can work independently or can be integrated into the ventilation apparatus as a module.

The diaphragm pacing apparatus is configured to contact a target position of a ventilated object through a stimulation electrode, and transmit a stimulation signal to the target position, so as to perform diaphragm pacing on the ventilated object. The Diaphragmatic pacing apparatus can provide stimulation either in an invasive manner or a non-invasive manner. Wherein, the invasive manner includes implanting a stimulation electrode inside a body of the ventilated object, or stimulating through an electrode needle inserted into a body surface of the ventilated object. The non-invasive manner includes an electrical stimulation through an electrode at the body surface, or a magnetic stimulation through an induction coil.

Exemplarily, the target position, which is stimulated by the diaphragm pacing apparatus, includes at least one of: a phrenic nerve, a diaphragm, an abdominal muscle. Wherein the diaphragm is the main respiratory muscle. The contraction of the diaphragm causes the diaphragm to descend, so as to expand a chest cavity in all directions, reduce an intrapulmonary pressure, and allow an external gas to enter into the lungs. When the diaphragm relaxes, a top of the diaphragm rises, a volume of the chest cavity decreases, and the lungs retract, thereby so as to discharge a gas from the lungs. The phrenic nerve is a nerve that controls a movement of the diaphragm. Stimulating the phrenic nerve can cause the diaphragm to contract. The abdominal muscle is also the main muscle group involved in spontaneous respiration, which can change an intra-abdominal pressure, so as to help to empty the lungs.

Exemplarily, when the phrenic nerve and the abdominal muscle are stimulated in a non-invasive manner, a stimulation electrode for phrenic nerve is configured to stimulate the phrenic nerve that controls the movement of the diaphragm, enable the diaphragm to contract and relax, a reference electrode for phrenic nerve is configured to form a loop with the stimulation electrode for phrenic nerve; a stimulation electrode for abdominal muscle is configured to stimulate the abdominal muscle, so as to enable the abdominal muscle to contract and relax, and a reference electrode for abdominal muscle is configured to form a loop with the stimulation electrode for abdominal muscle. It should be noted that, a position and a number of the stimulation electrode are just for example. The embodiments of this disclosure do not limit the specific structure of the diaphragm pacing apparatus.

The method 100 for controlling a ventilation apparatus in an embodiment of this disclosure can be implemented by a processor, which may be a processor of the ventilation apparatus, a processor of a diaphragm pacing apparatus, or a processor of another external apparatus which is in communicative connection with the ventilation apparatus and the diaphragm pacing apparatus. During the ventilation process, the processor is configured to obtain a ventilation target for providing mechanical ventilation to the ventilated object, and obtain a physiological state parameter which represents a physiological state of the ventilated object, as a basis for a coordinated control of mechanical ventilation and diaphragm pacing. According to the physiological state parameter which represents the physiological state of the ventilated object and the ventilation target of the mechanical ventilation, the processor is configured to set a ventilation parameter for the mechanical ventilation and an intensity of a stimulation signal for the diaphragm pacing, and complete a coordinated control on the ventilation apparatus and the diaphragm pacing apparatus, so as to ultimately achieve the ventilation target.

The physiological state parameter at least includes a monitoring parameter which is related to ventilation. The monitoring parameter which is related to ventilation can be acquired by a sensor of the ventilation apparatus. When the method 100 for controlling a ventilation apparatus is implemented by a processor of the ventilation apparatus, the processor of the ventilation apparatus may acquire a monitoring parameter which is related to ventilation through a sensor of the ventilation apparatus during ventilation. When the method 100 for controlling a ventilation apparatus is implemented by a processor of a diaphragm pacing apparatus or a third-party apparatus, the monitoring parameter which is related to ventilation can be acquired by a sensor of the ventilation apparatus and then obtained through communicative connection with the ventilation apparatus. Exemplarily, the monitoring parameter which is related to ventilation may include at least one of: a ventilation pressure parameter, a flow rate parameter, a volume parameter, and a time parameter, which are monitored, and may also include a parameter which is derived from at least one of : a ventilation pressure parameter, a flow rate parameter, a volume parameter, and a time parameter, such as a parameter which reflects a change characteristic of a pressure or a flow rate.

In addition to the monitoring parameter which is related to ventilation, the physiological state parameter may also include another parameter, which can reflect a physiological state of the ventilated object, such as at least one of: a blood oxygen parameter, a blood pressure parameter, a carbon dioxide parameter, a heart rate parameter, a diaphragmatic electricity parameter, a transdiaphragmatic pressure parameter, an esophageal pressure parameter, an electrical impedance parameter, an ultrasound parameter, and a blood-gas-related parameter. The above physiological state parameters can be acquired by the ventilation apparatus or from other medical apparatus that are in communicative connection with the processor. For example, parameters, such as the heart rate parameter, blood pressure parameter, and blood oxygen parameter can come from a monitor, and the ultrasound parameter can come from an ultrasound apparatus. These physiological parameters can indirectly reflect impacts of mechanical ventilation and diaphragm pacing on the physiological state of the ventilated object, thereby assisting in decision-making on the control of the mechanical ventilation and the diaphragm pacing.

Based on the acquired physiological state parameter and ventilation target of the ventilated object, the ventilation apparatus and the diaphragm pacing apparatus can be controlled in a coordinated way to achieve the ventilation target. Illustratively, the ventilation target for providing mechanical ventilation to a ventilated object includes a tidal volume target or an intensity target for spontaneous respiration. The tidal volume target is a tidal volume which is expected to achieve during ventilation, and the intensity target for spontaneous respiration is a spontaneous respiration intensity which is expected to achieve during ventilation. When the ventilation target is a tidal volume target, the tidal volume target is achieved by setting the intensity of the stimulation signal for the diaphragm pacing which signal is transmitted to the target position of the ventilated object, and by setting the ventilation parameter for the mechanical ventilation of the ventilated object. When the ventilation target is an intensity target for spontaneous respiration, the intensity target for spontaneous respiration is achieved by setting the intensity of the stimulation signal for the diaphragm pacing which signal is transmitted to the target position of the ventilated object, and by setting the ventilation parameter for the mechanical ventilation of the ventilated object.

Wherein, the ventilation parameter for the mechanical ventilation includes at least one of: a pressure parameter, a flow rate parameter, a volume parameter, a time parameter, a respiratory ratio parameter, a respiratory rate parameter, a trigger mode parameter, and a trigger sensitivity parameter, for the mechanical ventilation which is provided by the ventilation apparatus. The ventilation parameter can also include a parameter which is derived from at least one of: a pressure parameter, a flow rate parameter, a volume parameter, a time parameter, a respiratory ratio parameter, a respiratory rate parameter, a trigger mode parameter, and a trigger sensitivity parameter, for the mechanical ventilation which is provided by the ventilation apparatus. The ventilation parameter may also include a parameter which is related to a device that is capable of controlling a pressure parameter, a flow rate parameter, etc., or any other parameter that can affect the mechanical ventilation.

The intensity of the stimulation signal for the diaphragm pacing apparatus is related to a stimulation mode of the diaphragm pacing apparatus. Exemplarily, the diaphragm pacing apparatus stimulates the target position in a manner including at least one of: a current stimulation manner, a voltage stimulation manner, and a magnetic stimulation manner. When the current stimulation manner is used, the stimulation intensity is adjusted by adjusting a magnitude of a stimulation current; when a voltage stimulation manner is used, the stimulation intensity is adjusted by adjusting a magnitude of a stimulation voltage. Magnetic stimulation mainly acts on the nervous system through a pulsed magnetic field, so as to change a membrane potential of cortical nerve cells for generating an induced current and producing a neural electrical activity. When the magnetic stimulation manner is used, the intensity of the stimulation signal can be adjusted by adjusting an intensity of a pulsed magnetic field.

Exemplarily, when spontaneous respiration of the ventilated object is detected, the acquired physiological state parameter also includes a respiration parameter, which is related to the spontaneous respiration of the ventilated object, and used to reflect a spontaneous respiration state of the ventilated object. Respiratory parameter related to spontaneous respiration includes a pressure parameter, a flow rate parameter, etc. For example, a difference between flow rates in an inspiratory end and in an expiratory end can be used to determine whether the ventilated object has spontaneous respiration, or a change of pressure in the respiratory loop can be detected to determine whether the ventilated object has spontaneous respiration.

When determining that the ventilated object has spontaneous respiration, a spontaneous respiration cycle of the ventilated object can be determined based on the respiratory parameter which is related to the spontaneous respiration, and the intensity of the stimulation signal for the diaphragm pacing and the ventilation parameter for the mechanical ventilation can be controlled according to the spontaneous respiration cycle, so as to achieve the ventilation target. For example, when the ventilated object actively inhales, a pressure inside an airway decreases or a flow rate inside the airway changes. When detecting an inspiratory movement of the ventilated object, a gas delivery is started, and the diaphragm is stimulated to contract, so as to synchronize the active inhalation of the ventilated object, the gas delivery of the mechanical ventilation and the diaphragm pacing, thus avoiding human-machine confrontation, alleviating discomfort of the ventilated object, and avoiding possible complications. Since the embodiment of this disclosure control the mechanical ventilation and the diaphragm pacing in a coordinated way, the spontaneous respiration cycle, which is identified by the ventilation apparatus, can be used to control the diaphragm pacing, thereby ensuring the synchronization of the diaphragm pacing, the mechanical ventilation and the spontaneous respiration.

On the contrary, for a ventilated object without spontaneous respiration, when the spontaneous respiration of the ventilated object is not detected, the intensity of the stimulation signal for the diaphragm pacing and the ventilation parameter for the mechanical ventilation can be controlled according to a preset respiratory cycle, so as to achieve the ventilation target, and ensure the synchronization between the negative pressure ventilation of the diaphragm pacing and the positive pressure ventilation of the mechanical ventilation.

During diaphragmatic pacing, stimulation is applied at different positions during different phases of a respiratory cycle. Specifically, during an inspiratory phase of the respiratory cycle, a stimulation signal is transmitted to the diaphragm of the ventilated object; during an expiratory phase of the respiratory cycle, a stimulation signal is transmitted to an abdominal muscle of the ventilated object. It should be noted that transmitting a stimulation signal to the diaphragm of the ventilated object includes transmitting a stimulation signal to the diaphragm and transmitting a stimulation signal to the phrenic nerve. Specifically, a stimulation signal for phrenic nerve can be outputted to the stimulation electrode for phrenic nerve, so as to stimulate the phrenic nerve during an inspiratory phase, and a stimulation signal for abdominal muscle can be outputted to the stimulation electrode for abdominal muscle, so as to stimulate the abdominal muscle during an expiratory phase. The diaphragm of the ventilated object contracts under the stimulation of the stimulation electrode for phrenic nerve, thereby generating an inspiratory drive force and increasing an inspiratory tidal volume; the abdominal muscle of the ventilated object contracts under the stimulation of the stimulation electrode for abdominal muscle, thereby generating an expiratory drive force and increasing an expiratory flow rate.

During ventilation, the diaphragmatic pacing provides at least part of the respiratory drive through the stimulation manner as described above. The ventilation apparatus needs to at least provide a gas with a preset oxygen concentration and achieve a positive end-expiratory pressure, so as to make up for shortcomings that the diaphragmatic pacing cannot provide respiratory gases with different oxygen concentrations and cannot provide a positive end-expiratory pressure. The tidal volume of ventilation can be collectively achieved by the diaphragm pacing and the positive pressure ventilation, and a contribution ratio of the diaphragm pacing to the positive pressure ventilation can be set or configured arbitrarily. In some embodiments, the intensity of the stimulation signal for the diaphragm pacing which signal is transmitted to the target position of the ventilated object, and the ventilation parameter for the mechanical ventilation of the ventilated object can be calculated based on the physiological state parameter and the ventilation target, that is, the intensity of the stimulation signal and the ventilation parameter are both obtained through calculation. In other embodiments, either the intensity of the stimulation signal or the ventilation parameter can also be a fixed value.

In some embodiments, when setting the intensity of the stimulation signal for the diaphragm pacing which signal is transmitted to the target position of the ventilated object, and setting the ventilation parameter for the mechanical ventilation of the ventilated object, a closed-loop control can be performed on at least one of the intensity of the stimulation signal for the diaphragm pacing and the ventilation parameter for the mechanical ventilation, so as to achieve the ventilation target. Closed-loop control is to continuously detect a parameter which is related to the ventilation target, and continuously adjust the intensity of the stimulation signal and/or the ventilation parameter according to a deviation between the detected parameter and the ventilation target, so as to ensure that the ventilation target is continuously achieved during the ventilation. During the closed-loop control, the intensity of the stimulation signal and the ventilation parameter may be adjusted in a coordinated way to achieve the ventilation target. Alternatively, one of the intensity of the stimulation signal and the ventilation parameter may be adjusted.

For example, during ventilation, the ventilation parameter can be maintained unchanged, and the intensity of the stimulation signal can be adjusted to achieve the ventilation target. Wherein, maintaining the ventilation parameter unchanged may include maintaining the ventilation parameter at a value which is required to achieve a positive end-expiratory pressure, that is, the ventilation apparatus is only responsible for providing a respiratory gas with a preset oxygen concentration and ensuring a positive end-expiratory pressure, and the respiratory drive is entirely achieved by diaphragm pacing, thereby increasing the activity of the diaphragm. Alternatively, the ventilation parameter may be maintained at a value which is required to provide a respiratory drive, that is, the ventilation apparatus is responsible for providing a part of the respiratory drive, and a ratio of a respiratory drive which is borne by the diaphragm pacing to a respiratory drive which is borne by the ventilation apparatus may be set arbitrarily, and may be set automatically by the processor or according to a user instruction.

Alternatively, when setting the intensity of the stimulation signal for the diaphragm pacing and the ventilation parameter for the mechanical ventilation, the intensity of the stimulation signal for the diaphragm pacing may be maintained unchanged, and the ventilation target may be achieved by adjusting the ventilation parameter. In this embodiment, diaphragm pacing is responsible for providing a part of the respiratory drive, the ventilation apparatus is responsible for providing a respiratory gas, ensuring a positive end-expiratory pressure, and providing the remaining respiratory drive. The ventilation target, such as a tidal volume target or an intensity target for spontaneous respiration, is achieved by adjusting the ventilation parameter.

When setting the intensity of the stimulation signal for the diaphragm pacing and the ventilation parameter for the mechanical ventilation according to a physiological state parameter, an airway pressure of the ventilated object can be made lower than a preset pressure by adjusting the intensity of the stimulation signal and/or the ventilation parameter for mechanical ventilation. Reducing an airway pressure means reducing a thoracic pressure, which can avoid pulmonary pressure injury, and reduce an effect of the intrathoracic pressure on the heart, thus avoiding an increase of heart load or affection of circulation. The preset pressure may be a fixed pressure value, or the preset pressure may be adjusted according to the physiological state parameter of the ventilated object.

In summary, the method 100 for controlling a ventilation apparatus according to an embodiment of this disclosure combines diaphragm pacing with mechanical ventilation, and reduces a thoracic pressure through the diaphragm pacing while providing the mechanical ventilation, thereby reducing or avoiding lung injury or circulatory inhibition which is caused by the mechanical ventilation. Meanwhile, because the diaphragm also contracts to a certain extent, while receiving mechanical ventilation, diaphragm disuse atrophy and ventilator dependence can also be avoided.

On the other hand, an embodiment of this disclosure provides a ventilation system. Referring to FIG. 2, the ventilation system 200 includes a ventilation apparatus 210, a diaphragm pacing apparatus 220, and a processor 230. The ventilation apparatus 210 includes: a sensor which is configured to acquire a monitoring parameter which is related to ventilation; a respiratory loop, which is configured to deliver a gas which is provided by a gas source to a ventilated object for mechanical ventilation; a diaphragm pacing apparatus 220, which is configured to contact a target position of the ventilated object through a stimulation electrode, and transmit a stimulation signal to the target position, so as to perform diaphragm pacing on the ventilated object; a processor 230, which is connected with the ventilation apparatus 210 and the diaphragm pacing apparatus 220, and configured to set a ventilation parameter for the mechanical ventilation and an intensity of the stimulation signal for the diaphragm pacing. The ventilation system 200 may be used to implement the method 100 for controlling a ventilation apparatus described above. Only main functions of the ventilation system 200 are described below, and other specific details may be found above.

Exemplarily, the ventilation apparatus 210 can be implemented as a medical apparatus with a mechanical ventilation function, such as a ventilator, an anesthesia machine, an oxygen therapy machine, etc. The respiratory loop of the ventilation apparatus 210 is connected with a gas source interface and a ventilation control unit, and is configured to deliver a gas which is provided by the gas source interface to the ventilated object under a control of the ventilation control unit. The gas which is provided by the gas source interface to the ventilated object can be oxygen or a mixture of air and oxygen. The sensor includes but is not limited to a flow rate sensor for measuring a flow rate signal, and a pressure sensor for measuring a pressure signal.

Exemplarily, the stimulation electrode of the diaphragm pacing apparatus 220 is an invasive electrode or a non-invasive electrode, and the diaphragm pacing apparatus 220 stimulates the target position in at least one of following manners: a current stimulation manner, a voltage stimulation manner, and a magnetic stimulation manner. The target position, which is stimulated by the diaphragm pacing apparatus, includes at least one of: a phrenic nerve, a diaphragm, an abdominal muscle. Exemplarily, the processor 230 can control the diaphragm pacing apparatus 220 to transmit a stimulation signal to the diaphragm of the ventilated object during an inspiratory phase of the respiratory cycle, so as to stimulate the diaphragm to contract for providing inspiratory drive; and to transmit a stimulation signal to the abdominal muscle of the ventilated object during the expiratory phase of the respiratory cycle, so as to stimulate the abdominal muscle to contract for providing expiratory drive.

The processor 230 may be a processor of the ventilation apparatus 210, a processor of the diaphragm pacing apparatus 220, or a processor of a third-party apparatus that is in communicative connection with the ventilation apparatus 210 and the diaphragm pacing apparatus 220. The processor 230 may be implemented by software, hardware, firmware or any combination thereof, and may use circuit(s), single or multiple dedicated integrated circuits, single or multiple general purpose integrated circuits, single or multiple microprocessors, single or multiple programmable logic devices, or any combination of the foregoing circuits and/or devices, or other suitable circuit(s) or device(s). Moreover, the processor 230 can control other components in the ventilation apparatus 210, in the diaphragm pacing apparatus 220, or in the third-party apparatus, so as to perform desired functions.

In one embodiment, the ventilation system 200 also includes a communication module, which is configured to establish communicative connection between the ventilation apparatus 210 and the processor 230, and/or between the processor 230 and the diaphragm pacing apparatus 220, wherein the processor 230 controls the diaphragm pacing apparatus and/or the ventilation apparatus through the communicative connection. For example, when the processor 230 is the processor of the ventilation apparatus 210, the communication module 210 is configured to establish communicative connection between the processor 230 and the diaphragm pacing apparatus 220, and the processor 230 controls the diaphragm pacing apparatus 220 through the communicative connection. On the contrary, when the processor 230 is the processor of the diaphragm pacing apparatus 220, the communication module 210 is configured to establish communicative connection between the processor 230 and the ventilation apparatus 210, and the processor 230 controls the ventilation apparatus 210 through the communicative connection.

In some embodiments, the processor 230 controls the ventilation parameter for the mechanical ventilation and the intensity of the stimulation signal for the diaphragm pacing in following manner: acquiring a physiological state parameter which represents a physiological state of a ventilated object, wherein the physiological state parameter includes a ventilation-related monitoring parameter which is acquired by a sensor; acquiring a ventilation target for providing mechanical ventilation to the ventilated object; and setting an intensity of a stimulation signal for the diaphragm pacing which signal is transmitted to a target position of the ventilated object, and setting a ventilation parameter for the mechanical ventilation of the ventilated object, based on the physiological state parameter and the ventilation target.

Exemplarily, the monitoring parameter which is acquired by the sensor includes at least one of: a ventilation pressure parameter, a flow rate parameter, a volume parameter and/or a time parameter, which are/is monitored; and/or a parameter which is derived from at least one of : a ventilation pressure parameter, a flow rate parameter, a volume parameter and/or a time parameter. In addition to the monitoring parameter which is related to ventilation, the physiological state parameter also includes at least one of: a blood oxygen parameter, a blood pressure parameter, a carbon dioxide parameter, a heart rate parameter, a diaphragmatic electricity parameter, a transdiaphragmatic pressure parameter, an esophageal pressure parameter, an electrical impedance parameter, an ultrasound parameter, and a blood-gas-related parameter. The ventilation parameter for the mechanical ventilation of the ventilated object includes at least one of: a pressure parameter, a flow rate parameter, a volume parameter and/or a time parameter, a respiratory ratio parameter, a respiratory rate parameter, a trigger mode parameter, and a trigger sensitivity parameter, for the mechanical ventilation, which is/are provided by the ventilation apparatus; and/or parameter(s) which is(are) derived from at least one of: a pressure parameter, a flow rate parameter, a volume parameter and/or a time parameter, a respiratory ratio parameter, a respiratory rate parameter, a trigger mode parameter, and a trigger sensitivity parameter, for the mechanical ventilation, which is/are provided by the ventilation apparatus.

In some embodiments, the processor 230 calculates an intensity of the stimulation signal for the diaphragm pacing and/or the ventilation parameter for the mechanical ventilation according to the physiological state parameter and the ventilation target. Furthermore, the processor 230 may perform a closed-loop control on the intensity of the stimulation signal for the diaphragm pacing and/or the ventilation parameter for the mechanical ventilation, so as to achieve the ventilation target. For example, the processor 230 may perform a coordinated adjustment on the intensity of the stimulation signal and the ventilation parameter, so as to achieve the ventilation target.

When controlling the ventilation parameter for the mechanical ventilation and the stimulation intensity of diaphragmatic pacing, the processor 230 may maintain the ventilation parameter unchanged and achieve the ventilation target by adjusting the intensity of the stimulation signal. The ventilation parameter may be maintained at a value which is required to achieve a positive end-expiratory pressure, or the ventilation parameter may be maintained at a value which is required to provide a respiratory drive. Alternatively, the processor 230 may maintain the intensity of the stimulation signal unchanged and achieve the ventilation target by adjusting the ventilation parameter.

When mechanical ventilation is performed on the ventilated object, the processor 230 controls the ventilation parameter for the mechanical ventilation and the intensity of the stimulation signal for the diaphragm pacing, so as to enable the airway pressure of the ventilated object to be lower than a preset pressure, thus reducing lung injury which is caused by mechanical ventilation.

Exemplarily, the ventilation target includes a tidal volume target or an intensity target for spontaneous respiration. When a tidal volume target is used, the processor 230 achieves the desired tidal volume by controlling the ventilation parameter for the mechanical ventilation and the intensity of the stimulation signal for the diaphragm pacing; when an intensity target for spontaneous respiration is used, the processor 230 achieves the desired intensity target for spontaneous respiration by controlling the ventilation parameter for mechanical ventilation and the intensity of the stimulation signal for the diaphragm pacing.

When spontaneous respiration of the ventilated object is detected, a spontaneous respiration cycle of the ventilated object can be determined based on the respiratory parameter which is related to the spontaneous respiration; the intensity of the stimulation signal for the diaphragm pacing and the ventilation parameter for the mechanical ventilation can be controlled according to the spontaneous respiration cycle to achieve the ventilation target, thereby enhancing human-machine synchronization. When the spontaneous respiration of the ventilated object is not detected, the intensity of the stimulation signal for the diaphragm pacing and the ventilation parameter for the mechanical ventilation can be controlled according to a preset respiratory cycle to achieve the ventilation target.

The ventilation system 200 of the embodiment of this disclosure uses a processor to control the diaphragm pacing apparatus and the ventilation apparatus in a coordinated way, so as to reduce or avoid lung injury or circulatory inhibition which is caused by mechanical ventilation, and so as to prevent diaphragm disuse atrophy.

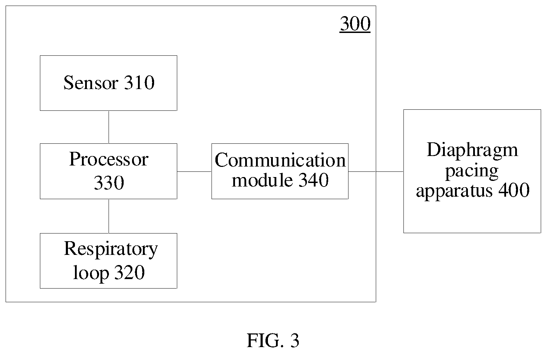

On the other hand, an embodiment of this disclosure provides a ventilation apparatus, which is capable of implementing the above-mentioned method 100 for controlling a ventilation apparatus. Continuing to refer to FIG. 3, a ventilation apparatus 300 according to an embodiment of this disclosure includes: a sensor 310, which is configured to acquire a monitoring parameter of a ventilated object which parameter is related to ventilation; a respiratory loop 320, which is configured to deliver a gas which is provided by a gas source to the ventilated object for mechanical ventilation; a communication module 340, which is capable of being in communicative connection with a diaphragm pacing apparatus 400, wherein the diaphragm pacing apparatus 400 is configured to contact a target position of the ventilated object through a stimulation electrode, and transmit a stimulation signal to the target position, so as to perform diaphragm pacing on the ventilated object; and a processor 330, which is configured to control a ventilation parameter for the mechanical ventilation and an intensity of the stimulation signal for the diaphragm pacing.

The ventilation apparatus 300 may be implemented as a medical apparatus with a mechanical ventilation function, such as a ventilator, an anesthesia machine, an oxygen therapy machine, etc. The communicative connection which is provided by the communication module 340 may be wired connection or wireless connection. The processor 330 controls an intensity of the stimulation signal for the diaphragm pacing which is provided by the diaphragm pacing apparatus 400 through the communicative connection which is provided by the communication module 340.

Exemplarily, the stimulation electrode of the diaphragm pacing apparatus 400 is an invasive electrode or a non-invasive electrode, and the diaphragm pacing apparatus 400 stimulates the target position in at least one of manners: a current stimulation manner, a voltage stimulation manner, and a magnetic stimulation manner. The target position, which is stimulated by the diaphragm pacing apparatus, includes at least one of: a phrenic nerve, a diaphragm, an abdominal muscle. Exemplarily, the processor 330 may control the diaphragm pacing apparatus 400 to transmit a stimulation signal to the diaphragm of the ventilated object during an inspiratory phase of the respiratory cycle, so as to stimulate the diaphragm to contract; and to transmit a stimulation signal to the abdominal muscle of the ventilated object during the expiratory phase of the respiratory cycle, so as to stimulate the abdominal muscle to contract.

In some embodiments, the processor 330 controls the ventilation parameter for mechanical ventilation and the intensity of the stimulation signal for the diaphragm pacing in following manner: acquiring a physiological state parameter which represents a physiological state of a ventilated object, wherein the physiological state parameter includes a ventilation-related monitoring parameter which is acquired by the sensor 310; acquiring a ventilation target for providing mechanical ventilation to the ventilated object; and setting an intensity of a stimulation signal for the diaphragm pacing whish signal is transmitted to a target position of the ventilated object, and setting a ventilation parameter for the mechanical ventilation of the ventilated object, based on the physiological state parameter and the ventilation target.

Exemplarily, the monitoring parameter which is acquired by the sensor 310 includes: a pressure parameter, a flow rate parameter, a volume parameter, and/or a time parameter, which are/is monitored during ventilation; and/or parameter(s) which is(are) derived from at least one of a pressure parameter, a flow rate parameter, a volume parameter, and/or a time parameter, which are/is monitored during ventilation. In addition to the monitoring parameter which is related to ventilation, the physiological state parameter also includes at least one of: a blood oxygen parameter, a blood pressure parameter, a carbon dioxide parameter, a heart rate parameter, a diaphragmatic electricity parameter, a transdiaphragmatic pressure parameter, an esophageal pressure parameter, an electrical impedance parameter, an ultrasound parameter, and a blood-gas-related parameter. The ventilation parameter for the mechanical ventilation of the ventilated object includes at least one of: a pressure parameter, a flow rate parameter, a volume parameter, and/or a time parameter, a respiratory ratio parameter, a respiratory rate parameter, a trigger mode parameter, and a trigger sensitivity parameter, for the mechanical ventilation, which is/are provided by the ventilation apparatus; and/or parameter(s) which is(are) derived from at least one of : a pressure parameter, a flow rate parameter, a volume parameter, and/or a time parameter, a respiratory ratio parameter, a respiratory rate parameter, a trigger mode parameter, and a trigger sensitivity parameter, for the mechanical ventilation, which is/are provided by the ventilation apparatus.

In some embodiments, the processor 330 calculates an intensity of the stimulation signal for the diaphragm pacing and/or the ventilation parameter for the mechanical ventilation, according to the physiological state parameter and the ventilation target. Furthermore, the processor 330 may perform a closed-loop control on the intensity of the stimulation signal for the diaphragm pacing and/or the ventilation parameter for the mechanical ventilation to achieve the ventilation target. For example, the processor 330 may perform a coordinated adjustment on the intensity of the stimulation signal and the ventilation parameter, so as to achieve the ventilation target.

When controlling the ventilation parameter for the mechanical ventilation and the stimulation intensity of diaphragmatic pacing, the processor 330 may maintain the ventilation parameter unchanged and achieve the ventilation target by adjusting the intensity of the stimulation signal. The ventilation parameter may be maintained at a value which is required to achieve a positive end-expiratory pressure, or the ventilation parameter may be maintained at a value which is required to provide a respiratory drive. Alternatively, the processor 330 may maintain the intensity of the stimulation signal unchanged and achieve the ventilation target by adjusting the ventilation parameter.

When mechanical ventilation is performed on the ventilated object, the processor 330 controls the ventilation parameter for the mechanical ventilation and the intensity of the stimulation signal for the diaphragm pacing, so as to enable the airway pressure of the ventilated object to be lower than a preset pressure, thus reducing lung injury which is caused by mechanical ventilation.

Exemplarily, the ventilation target includes a tidal volume target or an intensity target for spontaneous respiration. When a tidal volume target is adopted, the processor 330 achieves the expected tidal volume by controlling the ventilation parameter for the mechanical ventilation and the intensity of the stimulation signal for the diaphragm pacing; when an intensity target for spontaneous respiration is used, the processor 330 achieves the desired intensity target for spontaneous respiration by controlling the ventilation parameter for mechanical ventilation and the intensity of the stimulation signal for the diaphragm pacing.

When spontaneous respiration of the ventilated object is detected, a spontaneous respiration cycle of the ventilated object can be determined based on the respiratory parameter which is related to the spontaneous respiration; the intensity of the stimulation signal for the diaphragm pacing and the ventilation parameter for the mechanical ventilation can be controlled according to the spontaneous respiration cycle to achieve the ventilation target, thereby enhancing human-machine synchronization. When the spontaneous respiration of the ventilated object is not detected, the intensity of the stimulation signal for the diaphragm pacing and the ventilation parameter for the mechanical ventilation can be controlled according to a preset respiratory cycle to achieve the ventilation target.

The ventilation apparatus 300 of the embodiment of this disclosure uses a processor of the ventilation apparatus to control the diaphragm pacing apparatus and the ventilation apparatus in a coordinated way, so as to reduce or avoid lung injury or circulatory inhibition which is caused by mechanical ventilation, and so as to prevent diaphragm disuse atrophy.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, except mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A method for controlling a ventilation apparatus, **characterized in that**, comprising:
acquiring a physiological state parameter which represents a physiological state of a ventilated object, wherein the physiological state parameter comprises a monitoring parameter which is related to ventilation;
acquiring a ventilation target for providing mechanical ventilation to the ventilated object; and
setting an intensity of a stimulation signal for diaphragm pacing which signal is transmitted to a target position of the ventilated object and setting a ventilation parameter for the mechanical ventilation of the ventilated object, based on the physiological state parameter and the ventilation target, so as to perform the diaphragm pacing and the mechanical ventilation on the ventilated object.

2. The method according to claim 1, **characterized in that**, further comprising:
calculating the intensity of the stimulation signal for the diaphragm pacing which signal is transmitted to the target position of the ventilated object and/or calculating the ventilation parameter for the mechanical ventilation of the ventilated object, based on the physiological state parameter and the ventilation target.

3. The method according to claim 1, **characterized in that**, setting an intensity of a stimulation signal for diaphragm pacing which signal is transmitted to a target position of the ventilated object and setting a ventilation parameter for the mechanical ventilation of the ventilated object, comprise:
performing a closed-loop control on the intensity of the stimulation signal for the diaphragm pacing and/or the ventilation parameter for the mechanical ventilation, so as to achieve the ventilation target.

4. The method according to claim 1, **characterized in that**, setting an intensity of a stimulation signal for diaphragm pacing which signal is transmitted to a target position of the ventilated object and setting a ventilation parameter for the mechanical ventilation of the ventilated object, comprise:
maintaining the ventilation parameter unchanged, and achieving the ventilation target by adjusting the intensity of the stimulation signal.

5. The method according to claim 4, **characterized in that**, maintaining the ventilation parameter unchanged, comprises:
maintaining the ventilation parameter at a value which is required to achieve a positive end-expiratory pressure; or
maintaining the ventilation parameter at a value which is required to provide a respiratory drive.

6. The method according to claim 1, **characterized in that**, setting an intensity of a stimulation signal for diaphragm pacing which signal is transmitted to a target position of the ventilated object and setting a ventilation parameter for the mechanical ventilation of the ventilated object, comprise:
maintaining the intensity of the stimulation signal unchanged, and achieving the ventilation target by adjusting the ventilation parameter.

7. The method according to claim 3, **characterized in that**, performing a closed-loop control on the intensity of the stimulation signal for the diaphragm pacing and/or the ventilation parameter for the mechanical ventilation, so as to achieve the ventilation target, comprises:
performing a coordinated adjustment on the intensity of the stimulation signal and the ventilation parameter, so as to achieve the ventilation target.

8. The method according to any one of claims 1-7, **characterized in that**, further comprising:
enabling an airway pressure of the ventilated object to be lower than a preset pressure, when the mechanical ventilation is performed on the ventilated object.

9. The method according to any one of claims 1-8, **characterized in that**, the ventilation target comprises a tidal volume target or an intensity target for spontaneous respiration.

10. The method according to any one of claims 1-9, **characterized in that**, when spontaneous respiration of the ventilated object is detected, the physiological state parameter further comprises a respiratory parameter which is related to the spontaneous respiration of the ventilated object;
setting an intensity of a stimulation signal for diaphragm pacing which signal is transmitted to a target position of the ventilated object and setting a ventilation parameter for the mechanical ventilation of the ventilated object, comprise:
determining a spontaneous respiration cycle of the ventilated object according to the respiration parameter which is related to the spontaneous respiration; and controlling the intensity of the stimulation signal for the diaphragm pacing and the ventilation parameter for the mechanical ventilation according to the spontaneous respiration cycle, so as to achieve the ventilation target.

11. The method according to any one of claims 1-9, **characterized in that**, setting an intensity of a stimulation signal for diaphragm pacing which signal is transmitted to a target position of the ventilated object and setting a ventilation parameter for the mechanical ventilation of the ventilated object, comprise:
controlling the intensity of the stimulation signal for the diaphragm pacing and the ventilation parameter for the mechanical ventilation according to a preset respiration cycle, when spontaneous respiration of the ventilated object is not detected.

12. The method according to claim 10 or claim 11, **characterized in that**, transmitting the stimulation signal for the diaphragm pacing to the target position of the ventilated object, comprises:
transmitting the stimulation signal to a diaphragm of the ventilated object during an inspiratory phase of the respiratory cycle; and
transmitting the stimulation signal to an abdominal muscle of the ventilated object during an expiratory phase of the respiratory cycle.

13. The method according to claim 1, **characterized in that**, the monitoring parameter comprises: a pressure parameter, a flow rate parameter, a volume parameter, and/or a time parameter, which are/is monitored during ventilation; and/or the monitoring parameter comprises parameter(s), which is(are) derived from at least one of: a ventilation pressure parameter, a flow rate parameter, a volume parameter, and/or a time parameter, which are/is monitored during ventilation;
the physiological state parameter further comprises at least one of: a blood oxygen parameter, a blood pressure parameter, a carbon dioxide parameter, a heart rate parameter, a diaphragm electricity parameter, a transdiaphragmatic pressure parameter, an esophageal pressure parameter, an electrical impedance parameter, an ultrasound parameter, and a blood-gas parameter;
the ventilation parameter comprises at least one of: a pressure parameter, a flow rate parameter, a volume parameter and/or a time parameter, a respiratory ratio parameter, a respiratory rate parameter, a trigger mode parameter, and a trigger sensitivity parameter, for the mechanical ventilation which ventilation is provided by the ventilation apparatus; and/or the ventilation parameter comprises parameter(s) which is(are) derived from at least one of: a pressure parameter, a flow rate parameter, a volume parameter and/or a time parameter, a respiratory ratio parameter, a respiratory rate parameter, a trigger mode parameter, and a trigger sensitivity parameter, for the mechanical ventilation which ventilation is provided by the ventilation apparatus.

14. A ventilation system, **characterized in that**, comprising a ventilation apparatus, which comprises:
a sensor, which is configured to acquire a monitoring parameter of a ventilated object, which parameter is related to ventilation;
a respiratory loop, which is configured to deliver a gas which is provided by a gas source to the ventilated object for mechanical ventilation;
a diaphragm pacing apparatus, which is configured to contact a target position of the ventilated object through a stimulation electrode, and transmit a stimulation signal to the target position, so as to perform diaphragm pacing on the ventilated object; and
a processor, which is connected with the ventilation apparatus and the diaphragm pacing apparatus, and configured to set an intensity of the stimulation signal for the diaphragm pacing and a ventilation parameter for the mechanical ventilation.

15. The ventilation system according to claim 14, **characterized in that**, further comprising a communication module which is configured to establish communicative connection between the ventilation apparatus and the processor, and/or between the diaphragm pacing apparatus and the processor;
wherein the processor is further configured to control the diaphragm pacing apparatus and/or the ventilation apparatus through the communicative connection.

16. The ventilation system according to claim 14, **characterized in that**, the diaphragm pacing apparatus is a plug-in module, an external module or a built-in integrated module, of the ventilation apparatus; and/or
the diaphragm pacing apparatus is capable of independently contacting the target position of the ventilated object through the stimulation electrode, and transmitting the stimulation signal to the target position, so as to perform the diaphragm pacing on the ventilated object.

17. The ventilation system according to claim 14, **characterized in that**, the target position comprises at least one of: a phrenic nerve, a diaphragm, an abdominal muscle.

18. The ventilation system according to claim 14, **characterized in that**, the stimulation electrode is an invasive electrode or a non-invasive electrode.

19. The ventilation system according to claim 14, **characterized in that**, the diaphragm pacing apparatus is configured to stimulate the target position in at least one of following manners: current stimulation, voltage stimulation, and magnetic stimulation.

20. A ventilation apparatus, **characterized in that**, comprising
a sensor, which is configured to acquire a monitoring parameter of a ventilated object, which parameter is related to ventilation;
a respiratory loop, which is configured to deliver a gas which is provided by a gas source to the ventilated object for mechanical ventilation;
a communication module, which is capable of being in communicative connection with a diaphragm pacing apparatus; wherein the diaphragm pacing apparatus is configured to contact a target position of the ventilated object through a stimulation electrode, and transmit a stimulation signal to the target position, so as to perform diaphragm pacing on the ventilated object; and
a processor, which is configured to control a ventilation parameter for the mechanical ventilation and an intensity of the stimulation signal for the diaphragm pacing.
